(19) Europäisches Patentamt
European Patent Office
Office européen des brevets

(11) Veröffentlichungsnummer : **0 243 842 B1**

# (12) EUROPÄISCHE PATENTSCHRIFT

(45) Veröffentlichungstag der Patentschrift : **30.01.91 Patentblatt 91/05**

(51) Int. Cl.$^5$ : **C07D 233/61,** C07D 249/08, A01N 47/36

(21) Anmeldenummer : **87105795.6**

(22) Anmeldetag : **18.04.87**

## (54) Aryloxycarbamoylazole und diese enthaltende Fungizide.

Die Akte enthält technische Angaben, die nach dem Eingang der Anmeldung eingereicht wurden und die nicht in dieser Patentschrift enthalten sind.

(30) Priorität : **30.04.86 DE 3614608**

(43) Veröffentlichungstag der Anmeldung : **04.11.87 Patentblatt 87/45**

(45) Bekanntmachung des Hinweises auf die Patenterteilung : **30.01.91 Patentblatt 91/05**

(84) Benannte Vertragsstaaten : **AT BE CH DE ES FR GB GR IT LI NL SE**

(56) Entgegenhaltungen :
**EP-A- 0 175 188**
**DE-A- 3 415 138**
**GB-A- 1 469 772**
**GB-A- 2 011 414**

(56) Entgegenhaltungen :
**CHEMICAL ABSTRACTS; Band 92, Nr. 13, 31.März 1980, Columbus, Ohio, USA WAKERLEY, ST.B.: "Composition for controlling fungus by treating rice plants or seeds" Seite 213, Spalte 2, Zusammenfassung Nr. 105 835d**

(73) Patentinhaber : **BASF Aktiengesellschaft**
**Carl-Bosch-Strasse 38**
**D-6700 Ludwigshafen (DE)**

(72) Erfinder : **Rentzea, Costin, Dr.**
**Richard-Kuhn-Strasse 1-3**
**D-6900 Heidelberg (DE)**
Erfinder : **Sauter, Hubert, Dr.**
**Neckarpromenade 20**
**D-6800 Mannheim 1 (DE)**
Erfinder : **Ammermann, Eberhard, Dr.**
**Sachsenstrasse 3**
**D-6700 Ludwigshafen (DE)**
Erfinder : **Pommer, Ernst-Heinrich, Dr.**
**Berliner Platz 7**
**D-6703 Limburgerhof (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die vorliegende Erfindung betrifft neue Aryloxycarbamoylazole, Verfahren zu ihrer Herstellung und fungizide Mittel, die diese Verbindungen als Wirkstoffe enthalten.

Es ist bekannt, N-Isopropyl-N-phenoxy-ethyl-N'-imidazolyl-harnstoff als Fungizid zu verwenden. (GB-1 469 772).

Es wurde nun gefunden, daß Aryloxycarbamoylazole der Formel (I)

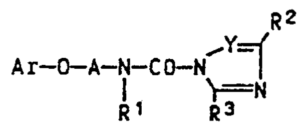
(I),

worin Ar Phenyl oder einen durch Halogen, Trifluormethyl, C1- bis $C_4$-Alkyl, $C_1$- bis $C_6$-Alkoxy, Nitro oder Cyano substituierten Phenylrest bedeutet, und

$R^1$ einen durch Fluor, Chlor oder Brom substituierten Alkylrest mit 1 bis 6 Kohlenstoffatomen bedeutet, und

A den Rest $-(CH_2)_n-$ mit n=1 bis 10 bedeutet, Y CH oder N bedeutet, und

$R^2$ und $R^3$ gleich oder verschieden sind und Wasserstoff oder Alkyl mit 1 bis 5 C-Atomen bedeuten, ausgezeichnet wirksam gegen Schadpilze sind.

Als Reste Ar in Formel (I) kommen Reste wie Phenyl, 1- bis 3-fach substituiertes Phenyl, 2-, 3- und 4-Fluorphenyl, 2-, 3- und 4-Chlorphenyl, 4-Bromphenyl, 4-Methylphenyl, 4-Ethylphenyl, 4-Isopropylphenyl, 4-tert.-Butylphenyl, 4-Isoamylphenyl, 2,4-, 2,5-, 2,6-, 3,4- und 3,5-Dithlorphenyl, 2,4-, 2,5-, 2,6-, 3,4- und 3,5-Dimethylphenyl, 4-Methoxyphenyl, 4-Ethoxyphenyl, 4-Propoxyphenyl, 4-Butoxyphenyl, 4-Hexyloxyphenyl, 4-Trifluormethylphenyl, 4-Nitrophenyl oder 2- oder 45-Cyanphenyl in Frage. Für $R^1$ kommen vorzugsweise Reste wie 1- bis 2-fach (vorzugsweise endständig) substituiertes $C_2$-$C_6$-Alkyl, insbesondere $C_2$-$C_3$-Alkyl, z.B. 2-Chlorethyl, 2-Bromethyl, 3-Chlorpropyl, 3-Brompropyl, 2,3-Dichlorpropyl, 2,3-Dibrompropyl, 4-Fluorbutyl, 4-Chlorbutyl, 5-Fluorpentyl, 5-Chlorpentyl oder 5-Brompentyl in Betracht. $R^2$ und $R^3$ bedeuten vorzugsweise Wasserstoff, Methyl, Ethyl, n-Propyl und Isopropyl. A ist beispielsweise -(CH2)n-, wobei n z.B. 2 bis 8, vorzugsweise 2 bis 6, ist.

Die Verbindungen der Formel (I) lassen sich herstellen, indem man ein Carbamoylchlorid der Formel (II)

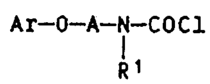
(II),

in der Ar, A und $R^1$ die oben angegebenen Bedeutungen haben,

a) mit Azolen der Formel (III)

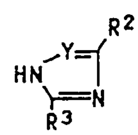
(III),

in der $R^2$, $R^3$ und Y die oben angegebenen Bedeutungen haben, oder

b) mit deren Metall-Derivaten der Formel (IV),

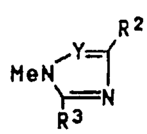
(IV),

in der $R^2$, $R^3$ und Y die oben angegebenen Bedeutungen haben und Me Lithium, Natrium, Kalium oder Calcium bedeutet, oder

c) mit deren Silyl-Derivaten der Formel (V)

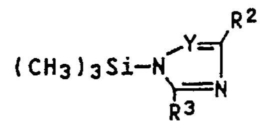 (V),

in der $R^2$, $R^3$ und Y die oben angegebenen Bedeutungen haben, umsetzt.

Die Reaktion a) erfolgt gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120°C .

Als bevorzugte, gegenüber den Reaktionsteilnehnern inerte Lösungs- oder Verdünnungsmittel können beispielsweise aliphatische oder aromatische, gegebenenfalls halogenierte Kohlenwasserstoffe, wie n-Pentan, Cyclohexan, Methylenchlorid, 1,1,1-Trichlorethan, Benzol, Toluol, Xylol, Chlorbenzol, aliphatische Ketone, wie Aceton, Methylethylketon, Diethylketon, Ether, wie Diethgylether, Methyl-tert.-butylether, Dimethoxyethan, Tetrahydrofuran oder Dioxan, Ester, wie Essigsäureethylester, Nitrile, wie Acetonitril, Amide, wie Dimethylformamid, Dimethylacetamid und N-Methylpyrrolidon oder Gemische dieser Lösungsmittel verwendet werden.

Geeignete Basen, die gegebenenfalls auch als säurebindende Mittel bei der Reaktion verwendet werden können, sind beispielsweise Alkalihydroxide, wie Lithium, Natrium-, Kalium- oder Calciumhydroxid, Alkalicarbonate, wie Natrium- oder Kaliumcarbonat oder Natrium- und Kaliumhydrogencarbonat, Amine, wie Triethylamin, Tripropylamin, N-Methylpyrrolidin, N-Methylpiperidin, N,N-Dimethylcyclohexylamin, N,N'-Tetramethylethylendiamin, N,N-Dimethylanilin, N,N-Diethylanilin, Pyridin oder 4-Dimethylaminopyridin. Es können aber auch andere übliche Basen verwendet werden.

Als Reaktionsbeschleuniger kommen vorzugsweise Metallhalogenide, wie Natriumiodid oder Kaliumiodid, quaternäre Ammoniumsalze, wie Tetrabutylammonium-chlorid, -bromid oder -iodid, Benzyltriethylammonium-chlorid oder -bromid oder Kronenether, wie 12-Krone-4, 15-Krone-5, 18-Krone-6 oder Dibenzo-18-krone-6 in Frage.

Die Reaktionen b) und c) erfolgen gegebenenfalls in Gegenwart eines Lösungs- oder Verdünnungsmittels bei einer Temperatur im Bereich zwischen 0 und 140°C, vorzugsweise zwischen 0 und 100°C. Hierfür eignen sich dieselben Lösungsmittel wie für die Verfahrensvariante a).

Die Verbindungen der Formel (I) lassen sich ferner herstellen, indem man ein sekundäres Amin der Formel (VI)

$$Ar{-}O{-}A{-}NH{-}R^1 \quad (VI),$$

in der Ar, $R^1$ und A die oben angegebenen Bedeutungen haben, mit einem Carbonyl-bisazol der Formel (VII)

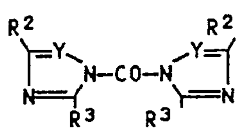 (VII),

in der $R^2$, $R^3$ und Y die oben angegebenen Bedeutungen haben, gegebenenfalls in Gegenwart eines Lösungs- oder Verdünnungsmittels, gegebenenfalls unter Zusatz eines Reaktionsbeschleunigers, umsetzt.

Hierfür eignen sich beispielsweise folgende Lösungs- oder Verdünnungsmittel : Diethylether, 1,2-Dimethoxyethan, Dipropylether, Dibutylether, Methyl-tert.-butylether, Tetrahydrofuran, Dimethoxyethan, Anisol, n-Pentan, n-Hexan, n-Heptan, n-Octan, Isooctan, Cyclohexan, Toluol, Chlorbenzol, Xylole, Acetonitril, Essigester, Dimethylformamid, N-Methylpyrrolidon, Aceton oder Methylethylketon.

Geeignete Reaktionsbeschleuniger sind beispielsweise 4-Dimethylaminopyridin oder 4-Pyrrolidinopyridin.

Die Ausgangsstoffe der Formel (II) können leicht nach bekannten Verfahren hergestellt werden, beispielsweise durch Umsetzung von Aminen der Formel (VI) mit Phosgen (Houben-Weyl-Müller, Methoden der organischen Chemie, Band 8, Seite 115 bis 118, Georg Thieme Verlag, Stuttgart 1952).

Die folgenden Beispiele erläutern die Herstellung der Verbindungen der Formel (I) :

Beispiel 1

a) Zu 130 g Pyrrolidon werden bei 25°C 45,8 (0,2 Mol) 4-Phenoxybutylbromid in 2 Stunden unter Rühren getropft. Nach 14-stündigem Rühren bei 25°C wird das Gemisch auf 10°C abgekühlt und nacheinander mit 200 ml Diethylether und 20% iger Natronlauge versetzt. Die organische Schicht wird abgetrennt, über $Na_2SO_4$ getrocknet und im Vakuum fraktioniert destilliert. Man erhält 32 g N-(4-Phenoxybutyl)-pyrrolidin als farblose Flüssigkeit vom Siedepunkt 123°C 0,1 mbar und $n_D^{24}$ 1,5193.

b) Unter gleichzeitiger Phosgeneinleitung werden 100 ml Essigester bei+10°C mit 26 g (0,118 Mol) N-(4-Phenoxybutyl)-pyrrolidin tropfenweise versetzt. Das Gemisch wird anschließend bei 45°C abgekühlt und unter vermindertem Druck eingeengt. Man erhält 32 g N-Chlorcarbonyl-N-(4-chlorbutyl)-N-(4-phenoxybutyl)-amin als farbloses Öl, das weiter umgesetzt wird.

c) Zu einer Lösung von 8,3 g (0,12 Mol) Imidazol in 100 ml trockenem Tetrahydrofuran werden bei 25°C 16 g (0,05 Mol) N-Chlorocarbonyl-N-(4-chlorbutyl)-N-(4-phenoxybutyl)-amin zugetropft. Nach 6-stündigem Rühren bei 70°C wird das Gemisch auf 20°C abgekühlt und der gebildete Niederschlag abgesaugt. Das Filtrat wird im Vakuum eingeengt, der Rückstand in 200 ml Methylenchlorid aufgenommen, dreimal mit je 60 ml Wasser gewaschen, getrocknet und eingeengt. Man erhält 15,6 g 1-[N-(4-phenoxybutyl)-N-(4-chlorbutyl)]-carbamoylimidazol als farbloses Öl vom n $n_D^{23}$ 1,5531 (Verbindung Nr. 1).

Entsprechend können durch Wahl der Ausgangsstoffe und entsprechende Anpassung der Verfahrensbedingungen die nachstehend aufgelisteten Verbindungen erhalten werden :

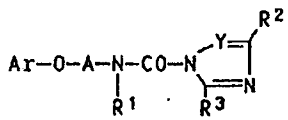

| Beisp. Nr. | Ar | A | $R^1$ | Y | $R^2$ | $R^3$ | IR-Spektrum [$cm^{-1}$] oder Phys.Konstante |
|---|---|---|---|---|---|---|---|
| 2 | $C_6H_5$- | $-(CH_2)_4-$ | $-(CH_2)_4Cl$ | N | H | H | $n_D^{22}$ 1.5427 |
| 3 | $C_6H_5$- | $-(CH_2)_2-$ | $-(CH_2)_3Cl$ | CH | H | H | Öl |
| 4 | $C_6H_5$- | $-(CH_2)_3-$ | $-(CH_2)_3Cl$ | CH | H | H | Öl |
| 5 | $C_6H_5$- | $-(CH_2)_3-$ | $-(CH_2)_3Cl$ | N | H | H | Öl |
| 6 | $C_6H_5$- | $-(CH_2)_3-$ | $-(CH_2)_4Cl$ | CH | H | H | Öl |
| 7 | $C_6H_5$- | $-(CH_2)_3-$ | $-(CH_2)_4Cl$ | N | H | H | Öl |
| 8 | $C_6H_5$- | $-(CH_2)_3-$ | $-(CH_2)_4Cl$ | CH | H | $CH_3$ | Öl |
| 9 | $C_6H_5$- | $-(CH_2)_3-$ | $-(CH_2)_4Cl$ | CH | $CH_3$ | $CH_3$ | Öl |
| 10 | $C_6H_5$- | $-(CH_2)_3-$ | $-(CH_2)_4Cl$ | CH | H | $C_2H_5$ | Öl |
| 11 | $C_6H_5$- | $-(CH_2)_3-$ | $-(CH_2)_4F$ | CH | H | H | Öl |
| 12 | $C_6H_5$- | $-(CH_2)_3-$ | $-(CH_2)_4F$ | N | H | H | Öl |
| 13 | $C_6H_5$- | $-(CH_2)_4-$ | $-(CH_2)_4Cl$ | CH | H | H | Öl |
| 14 | $C_6H_5$- | $-(CH_2)_4-$ | $-(CH_2)_4Cl$ | N | H | H | Öl |
| 15 | $C_6H_5$- | $-(CH_2)_4-$ | $-(CH_2)_4Cl$ | CH | H | $CH_3$ | Öl |
| 16 | $C_6H_5$- | $-(CH_2)_4-$ | $-(CH_2)_4Br$ | CH | H | H | Öl |
| 17 | $C_6H_5$- | $-(CH_2)_4-$ | $-(CH_2)_4Br$ | N | H | H | Öl |
| 18 | $C_6H_5$- | $-(CH_2)_5-$ | $-(CH_2)_4Cl$ | CH | H | H | $n_D^{23}$ 1.5450 |
| 19 | $C_6H_5$- | $-(CH_2)_5-$ | $-(CH_2)_4Cl$ | N | H | H | $n_D^{23}$ 1.5365 |

| Beisp. Nr. | Ar | A | $R^1$ | Y | $R^2$ | $R^3$ | IR-Spektrum [$cm^{-1}$] oder Phys. Konstante |
|---|---|---|---|---|---|---|---|
| 20 | $C_6H_5$- | $-(CH_2)_5-$ | $-(CH_2)_5Cl$ | CH | H | H | Öl |
| 21 | $C_6H_5$- | $-(CH_2)_5-$ | $-(CH_2)_5Cl$ | N | H | H | Öl |
| 22 | $C_6H_5$- | $-(CH_2)_6-$ | $-(CH_2)_4Cl$ | CH | H | H | $n_D^{23}$ 1.5409 |
| 23 | $C_6H_5$- | $-(CH_2)_6-$ | $-(CH_2)_4Cl$ | N | H | H | $n_D^{23}$ 1.5351 |
| 24 | $C_6H_5$- | $-(CH_2)_8-$ | $-(CH_2)_3Cl$ | CH | H | H | Öl |
| 25 | $C_6H_5$- | $-(CH_2)_8-$ | $-(CH_2)_3Cl$ | N | H | H | Öl |
| 26 | $C_6H_5$- | $-(CH_2)_8-$ | $-(CH_2)_4Cl$ | CH | H | H | Öl |
| 27 | 2-$FC_6H_4$- | $-(CH_2)_2-$ | $-(CH_2)_4Cl$ | CH | H | H | $n_D^{23}$ 1.5502 |
| 28 | 2-$FC_6H_4$- | $-(CH_2)_2-$ | $-(CH_2)_4Cl$ | N | H | H | $n_D^{23}$ 1.5378 |
| 29 | 2-$FC_6H_4$- | $-(CH_2)_4-$ | $-(CH_2)_4Cl$ | CH | H | H | $n_D^{22}$ 1.5426 |
| 30 | 2-$FC_6H_4$- | $-(CH_2)_4-$ | $-(CH_2)_4Cl$ | N | H | H | $n_D^{22}$ 1.5343 |
| 31 | 2-$FC_6H_4$- | $-(CH_2)_4-$ | $-CH_2-CH(Br)CH_2Br$ | CH | H | H | |
| 32 | 2-$FC_6H_4$- | $-(CH_2)_5-$ | $-(CH_2)_4Cl$ | CH | H | H | $n_D^{23}$ 1.5381 |
| 33 | 2-$FC_6H_4$- | $-(CH_2)_5-$ | $-(CH_2)_4Cl$ | N | H | H | $n_D^{23}$ 1.5230 |

| Beisp. Nr. | Ar | A | $R^1$ | Y | $R^2$ | $R^3$ | IR-Spektrum [$cm^{-1}$] oder Phys. Konstante |
|---|---|---|---|---|---|---|---|
| 34 | $4-FC_6H_4-$ | $-(CH_2)_2-$ | $-(CH_2)_2Cl$ | CH | H | H | Öl |
| 35 | $4-FC_6H_4-$ | $-(CH_2)_2-$ | $-(CH_2)_2Cl$ | N | H | H | Öl |
| 36 | $4-FC_6H_4-$ | $-(CH_2)_2-$ | $-(CH_2)_3Cl$ | CH | H | H | Öl |
| 37 | $4-FC_6H_4-$ | $-(CH_2)_2-$ | $-(CH_2)_3Br$ | CH | H | H | Öl |
| 38 | $4-FC_6H_4-$ | $-(CH_2)_2-$ | $-(CH_2)_4Cl$ | CH | H | H | Öl |
| 39 | $4-FC_6H_4-$ | $-(CH_2)_2-$ | $-(CH_2)_4Cl$ | CH | H | $CH_3$ | Öl |
| 40 | $4-FC_6H_4-$ | $-(CH_2)_4-$ | $-(CH_2)_4Cl$ | CH | H | H | $n_D^{22}$ 1.5362 |
| 41 | $4-FC_6H_4-$ | $-(CH_2)_5-$ | $-(CH_2)_4Cl$ | CH | H | H | Öl |
| 42 | $4-FC_6H_4-$ | $-(CH_2)_6-$ | $-(CH_2)_4Cl$ | CH | H | H | Öl |
| 43 | $2-ClC_6H_4-$ | $-(CH_2)_2-$ | $-(CH_2)_4Cl$ | CH | H | H | $n_D^{23}$ 1.5651 |
| 44 | $2-ClC_6H_4-$ | $-(CH_2)_2-$ | $-(CH_2)_4Cl$ | N | H | H | $n_D^{23}$ 1.5575 |
| 45 | $1-ClC_6H_4-$ | $-(CH_2)_4-$ | $-(CH_2)_4Cl$ | CH | H | H | $n_D^{24}$ 1.5532 |
| 46 | $2-ClC_6H_4-$ | $-(CH_2)_4-$ | $-(CH_2)_4Cl$ | N | H | H | $n_D^{24}$ 1.5482 |
| 47 | $2-ClC_6H_4-$ | $-(CH_2)_5-$ | $-(CH_2)_4Cl$ | CH | H | H | Öl |
| 48 | $2-ClC_6H_4-$ | $-(CH_2)_5-$ | $-(CH_2)_4F$ | CH | H | H | Öl |
| 49 | $2-ClC_6H_4-$ | $-(CH_2)_5-$ | $-(CH_2)_5Cl$ | CH | H | H | Öl |
| 50 | $2-Cl_6H_4-$ | $-(CH_2)_6-$ | $-(CH_2)_4Cl$ | CH | H | H | Öl |
| 51 | $4-ClC_6H_4-$ | $-(CH_2)_2-$ | $-(CH_2)_4Cl$ | CH | H | H | $n_D^{23}$ 1.5641 |

| Beisp. Nr. | Ar | A | $R^1$ | Y | $R^2$ | $R^3$ | IR-Spektrum [$cm^{-1}$] oder Phys.Konstante |
|---|---|---|---|---|---|---|---|
| 52 | $4-Cl_6H_4-$ | $-(CH_2)_2-$ | $-(CH_2)_4Cl$ | N | H | H | $n_D^{23}$ 1.5578 |
| 53 | $4-ClC_6H_4-$ | $-(CH_2)_2-$ | $-CH_2CH(Br)CH_2Br$ | CH | H | H | |
| 54 | $4-ClC_6H_4-$ | $-(CH_2)_2-$ | $-CH_2CH(Br)CH_2Br$ | CH | H | H | |
| 55 | $4-ClC_6H_4-$ | $-(CH_2)_4-$ | $-(CH_2)_4Cl$ | CH | H | H | Öl |
| 56 | $4-ClC_6H_4-$ | $-(CH_2)_5-$ | $-(CH_2)_4Cl$ | CH | H | H | $n_D^{23}$ 1.5465 |
| 57 | $4-ClC_6H_4-$ | $-(CH_2)_5-$ | $-(CH_2)_4Cl$ | N | H | H | $n_D^{23}$ 1.5410 |
| 58 | $4-ClC_6H_4-$ | $-(CH_2)_6-$ | $-(CH_2)_4Cl$ | CH | H | H | $n_D^{23}$ 1.5457 |
| 59 | $4-ClC_6H_4-$ | $-(CH_2)_6-$ | $-(CH_2)_4Cl$ | N | H | H | $n_D^{23}$ 1.5380 |
| 60 | $4-ClC_6H_4-$ | $-(CH_2)_7-$ | $-(CH_2)_4Cl$ | CH | H | H | $n_D^{23}$ 1.5430 |
| 61 | $4-ClC_6H_4-$ | $-(CH_2)_7-$ | $-(CH_2)_4Cl$ | N | H | H | $n_D^{23}$ 1.5330 |
| 62 | $4-ClC_6H_4-$ | $-(CH_2)_8-$ | $-(CH_2)_4Cl$ | CH | H | H | $n_D^{23}$ 1.5391 |
| 63 | $4-ClC_6H_4-$ | $-(CH_2)_8-$ | $-(CH_2)_4Cl$ | N | H | H | $n_D^{23}$ 1.5338 |
| 64 | $4-ClC_6H_4-$ | $-(CH_2)_9-$ | $-(CH_2)_4Cl$ | CH | H | H | Öl |

| Beisp. Nr. | Ar | A | $R^1$ | Y | $R^2$ | $R^3$ | IR-Spektrum [$cm^{-1}$] oder Phys.Konstante |
|---|---|---|---|---|---|---|---|
| 65 | $4-ClC_6H_4-$ | $-(CH_2)_{10}-$ | $-(CH_2)_4Cl$ | CH | H | H | Öl |
| 66 | $2-(CH_3)C_6H_4-$ | $-(CH_2)_2-$ | $-(CH_2)_4Cl$ | CH | H | H | Öl |
| 67 | $2-(CH_3)C_6H_4-$ | $-(CH_2)_3-$ | $-(CH_2)_4Cl$ | CH | H | H | Öl |
| 68 | $2-(CH_3)C_6H_4-$ | $-(CH_2)_4-$ | $-(CH_2)_4Cl$ | CH | H | H | $n_D^{23}$ 1.5465 |
| 69 | $2-(CH_3)C_6H_4-$ | $-(CH_2)_4-$ | $-(CH_2)_4Cl$ | N | H | H | $n_D^{22}$ 1.5391 |
| 70 | $2-(CH_3O)C_6H_4-$ | $-(CH_2)_2-$ | $-(CH_2)_4Cl$ | CH | H | H | Öl |
| 71 | $2-(CH_3O)C_6H_4-$ | $-(CH_2)_3-$ | $-(CH_2)_4Cl$ | CH | H | H | $n_D^{22}$ 1.5508 |
| 72 | $2-(CH_3O)C_6H_4-$ | $-(CH_2)_4-$ | $-(CH_2)_4Cl$ | CH | H | H | $n_D^{23}$ 1.5498 |
| 73 | $2-(CH_3O)C_6H_4-$ | $-(CH_2)_4-$ | $-(CH_2)_4Cl$ | N | H | H | $n_D^{23}$ 1.5432 |
| 74 | $4-(CH_3)C_6H_4-$ | $-(CH_2)_2-$ | $-(CH_2)_4Cl$ | CH | H | H | Öl |
| 75 | $4-(CH_3)C_6H_4-$ | $-(CH_2)_4-$ | $-(CH_2)_4Cl$ | CH | H | H | Öl |
| 76 | $4-(CH_3)C_6H_4-$ | $-(CH_2)_6-$ | $-(CH_2)_4Cl$ | CH | H | H | Öl |
| 77 | $4-(CF_3)C_6H_4-$ | $-(CH_2)_2-$ | $-(CH_2)_4Cl$ | CH | H | H | Öl |
| 78 | $4-(CF_3)C_6H_4-$ | $-(CH_2)_4-$ | $-(CH_2)_4Cl$ | CH | H | H | Öl |
| 79 | $4-(CF_3)C_6H_4-$ | $-(CH_2)_6-$ | $-(CH_2)_4Cl$ | CH | H | H | Öl |
| 80 | $4-[(CH_3)_3C]C_6H_4-$ | $-(CH_2)_2-$ | $-(CH_2)_4Cl$ | CH | H | H | $n_D^{22}$ 1.5380 |

| Beisp. Nr. | Ar | A | $R^1$ | Y | $R^2$ | $R^3$ | IR-Spektrum [$cm^{-1}$] oder Phys. Konstante |
|---|---|---|---|---|---|---|---|
| 81 | $4-[(CH_3)_3C]C_6H_4-$ | $-(CH_2)_2-$ | $-(CH_2)_4Cl$ | N | H | H | $n_D^{22}$ 1.5321 |
| 82 | $2,4-(CH_3)_2C_6H_3-$ | $-(CH_2)_2-$ | $-(CH_2)_4Cl$ | CH | H | H | Öl |
| 83 | $2,4-(CH_3)_2C_6H_3-$ | $-(CH_2)_4-$ | $-(CH_2)_4Cl$ | CH | H | H | Öl |
| 84 | $2,6-(CH_3)_2C_6H_3-$ | $-(CH_2)_2-$ | $-(CH_2)_4Cl$ | CH | H | H | Öl |
| 85 | $2,6-(CH_3)_3C_6H_3$ | $-(CH_2)_4-$ | $-(CH_2)_4Cl$ | CH | H | H | Öl |
| 86 | $2,4-(Cl_2C_6H_3-$ | $-(CH_2)_2-$ | $-(CH_2)_4Cl$ | CH | H | H | |
| 87 | $2,4-Cl_2C_6H_3-$ | $-(CH_2)_2-$ | $-CH_2CH(Br)CH_2Br$ | CH | H | H | |
| 88 | $2,4-Cl_2C_6H_3-$ | $-(CH_2)_2-$ | $-(CH_2)_4Cl$ | N | H | H | Fp. 71 - 74°C |
| 89 | $2,4-Cl_2C_6H_3-$ | $-(CH_2)_2-$ | $-CH_2CH(Br)CH_2Br$ | N | H | H | |
| 90 | $2,4-Cl_2C_6H_3-$ | $-(CH_2)_4-$ | $-(CH_2)_4Cl$ | CH | H | H | Öl |
| 91 | $2,6-Cl_2C_6H_3-$ | $-(CH_2)_2-$ | $-(CH_2)_4Cl$ | CH | H | H | $n_D^{23}$ 1.5610 |
| 92 | $2,6-Cl_2C_6H_3-$ | $-(CH_2)_2-$ | $-(CH_2)_4Cl$ | N | H | H | $n_D^{23}$ 1.5547 |
| 93 | $2,6-Cl_2C_6H_3-$ | $-(CH_2)_2-$ | $-CH_2CH(Br)CH_2Br$ | N | H | H | |
| 94 | $2,6-Cl_2C_6H_3-$ | $-(CH_2)_3-$ | $-(CH_2)_4Cl$ | CH | H | H | Fp. 55 - 56°C |
| 95 | $2,6-Cl_2C_6H_3-$ | $-(CH_2)_3-$ | $-(CH_2)_4Cl$ | N | H | H | Fp. 97 - 99°C |
| 96 | $2,6-Cl_2C_6H_3-$ | $-(CH_2)_4-$ | $-(CH_2)_4Cl$ | CH | H | H | Öl |
| 97 | $2,6-Cl_2C_6H_3-$ | $-(CH_2)_5-$ | $-(CH_2)_4Cl$ | CH | H | H | Öl |
| 98 | $2,6-Cl_2C_6H_3-$ | $-(CH_2)_6-$ | $-(CH_2)_4Cl$ | CH | H | H | $n_D^{23}$ 1.5488 |
| 99 | $2,6-Cl_2C_6H_3-$ | $-(CH_2)_2-$ | $-(CH_2)_4Cl$ | N | H | H | $n_D^{24}$ 1.5426 |

| Beisp. Nr. | Ar | A | $R^1$ | Y | $R^2$ | $R^3$ | IR-Spektrum [$cm^{-1}$] oder Phys. Konstante |
|---|---|---|---|---|---|---|---|
| 100 | 2,6-$Cl_2C_6H_3$- | -$(CH_2)_7$- | -$(CH_2)_4Cl$ | CH | H | H | Öl |
| 101 | 2,6-$Cl_2C_6H_3$- | -$(CH_2)_8$- | -$(CH_2)_4Cl$ | CH | H | H | Öl |
| 102 | 2,4,6-$(CH_3)_3C_6H_2$- | -$(CH_2)_2$- | -$(CH_2)_4Cl$ | CH | H | H | Öl |
| 103 | 2,4,6-$(CH_3)_3C_6H_2$- | -$(CH_2)_3$- | -$(CH_2)_4Cl$ | CH | H | H | $n_D^{23}$ 1.5430 |
| 104 | 2,4,6-$(CH_3)_3C_6H_2$- | -$(CH_2)_3$- | -$(CH_2)_4Cl$ | N | H | H | $n_D^{23}$ 1.5360 |
| 105 | 2,4,6-$(CH_3)_3C_6H_2$- | -$(CH_2)_4$- | -$(CH_2)_4Cl$ | CH | H | H | Öl |
| 106 | 2,4,6-$(CH_3)_3C_6H_2$- | -$(CH_2)_6$- | -$(CH_2)_4Cl$ | CH | H | H | Öl |
| 107 | 2,4-$Cl_2$-6-$(CH_3)C_6H_2$- | -$(CH_2)_2$- | -$(CH_2)_4Cl$ | CH | H | H | $n_D^{23}$ 1.5599 |
| 108 | 2,4-$Cl_2$-6-$(CH_3)C_6H_2$- | -$(CH_2)_2$- | -$(CH_2)_4Cl$ | N | H | H | $n_D^{20}$ 1.5540 |
| 109 | 2,4-$Cl_2$-6-$(CH_3)C_6H_2$- | -$(CH_2)_4$- | -$(CH_2)_4Cl$ | CH | H | H | Öl |
| 110 | 2,4-$Cl_2$-6-$(CH_3)C_6H_2$- | -$(CH_2)_5$- | -$(CH_2)_4Cl$ | CH | H | H | Öl |
| 111 | 2,4-$Cl_2$-6-$(CH_3)C_6H_2$- | -$(CH_2)_6$- | -$(CH_2)_4Cl$ | CH | H | H | Öl |
| 112 | 2,4,6-$Cl_3C_6H_2$- | -$(CH_2)_2$- | -$(CH_2)_4Cl$ | CH | H | H | Fp. 79 - 81°C |
| 113 | 2,4,6-$Cl_3C_6H_2$- | -$(CH_2)_2$- | -$(CH_2)_4Cl$ | N | H | H | $n_D^{22}$ 1.5660 |
| 114 | 2,4,6-$Cl_3C_6H_2$- | -$(CH_2)_2$- | -$CH_2CH(Br)CH_2Br$ | CH | H | H | |
| 115 | 2,4,6-$Cl_3C_6H_2$- | -$(CH_2)_2$- | -$CH_2CH(Br)CH_2Br$ | N | H | H | |
| 116 | 2,4,6-$Cl_3C_6H_2$- | -$(CH_2)_3$- | -$(CH_2)_4Cl$ | CH | H | H | Öl |
| 117 | 2,4,6-$Cl_3C_6H_2$- | -$(CH_2)_4$- | -$(CH_2)_3Cl$ | CH | H | H | Öl |

| Beisp. Nr. | Ar | A | $R^1$ | Y | $R^2$ | $R^3$ | IR-Spektrum [$cm^{-1}$] oder Phys.Konstante |
|---|---|---|---|---|---|---|---|
| 118 | 2,4,6-$Cl_3C_6H_2$- | -$(CH_2)_4$- | -$(CH_2)_4Cl$ | CH | H | H | Öl |
| 119 | 2,4,6-$Cl_3C_6H_2$- | -$(CH_2)_5$- | -$(CH_2)_4Cl$ | CH | H | H | Öl |
| 120 | 2,4,6-$Cl_3C_6H_2$- | -$(CH_2)_6$- | -$(CH_2)_4Cl$ | CH | H | H | Öl |
| 121 | 2,4,6-$Cl_3C_6H_2$- | -$(CH_2)_4$- | -$(CH_2)_4Cl$ | CH | H | H | Öl |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse – wie Gurken, Bohnen und Kürbisgewächse –.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle,
Ustilago-Arten an Getreide und Zuckerrohr,
Venturia inaequalis (Schorf) an Äpfeln,
Septoria nodorum an Weizen,
Helminthosporium teres an Gerste,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Alternaria solani an Kartoffeln, Tomaten,
Plasmopara viticola an Reben sowie
Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser ; Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden. Bei der Anwendung der Wirkstoffe im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind :

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.
II. 20 Gew.-Teile der Verbindung Nr. 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.
III. 20 Gew.-Teile der Verbindung Nr. 18 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.
IV. 20 Gew.-Teile der Verbindung Nr. 19 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.
V. 80 Gew.-Teile der Verbindung Nr. 27 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.
VI. 3 Gew.-Teile der Verbindung Nr. 29 werden mit 97 Gew.-Teilen feinteiligem Kaolin innig vermischt. Man erhält auf diese Weise ein Stäubemittel, das 3 Gew.% des Wirkstoffs enthält.
VII. 30 Gew.-Teile der Verbindung Nr. 32 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 40 Gew.-Teile der Verbindung Nr. 33 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.
IX. 20 Gew.-Teile der Verbindung Nr. 40 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise :

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid ;

Nitroderivate, wie

Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester

heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,

N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
1-(4-Phenylphenoxy)-3,3-dimethyl-1-(1H)-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,

Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4'-Difluor-α-(1H-1,2,4-triazolyl-1-methyl)-benzhydrylalkohol
N-(3-Chlor, 2,6-dinitro, 4-trifluormethylphenyl-5-trifluormethyl,
3-chlor, 2-aminopyridin,
1-((bis(4-Fluorphenyl)methylsilyl)-methyl-1H-1,2,4-triazol.

Für die folgenden Versuche wurde als bekanntes Vergleichsmittel das Fungizid N-Isopropyl-N-phenoxy-ethyl-N'-imidazolylharnstoff verwendet.

Anwendungsbeispiel 1

Wirksamkeit gegen Weizenmehltau

Blätter von in Töpfen gewachsenen Weizenkeimlingen der Sorte Frühgold werden mit wäßriger Spritzbrühe, die 80% Wirkstoff und 20% Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80% relativer Luftfeuchtigkeit aufgestellt. Nach 7 Tagen wird das Ausmaß der Mehltauentwicklung ermittelt.

Das Ergebnis des Versuches zeigt, daß die Wirkstoffe 1, 2, 18, 19, 27, 29, 32, 33, 40, 43, 44, 51, 53, 56, 57, 58, 59, 60, 61, 62, 63, 68, 69, 72, 80, 91, 92, 103, 104, 107, 108, 113 und 114 bei der Anwendung als 0,025% ige Spritzbrühe eine bessere fungizide Wirkung zeigen (97%) als das Vergleichsmittel (60%).

Anwendungsbeispiel 2

Wirksamkeit gegen Weizenbraunrost

Blätter von in Töpfen gewachsenen Weizensämlingen der Sorte "Frühgold" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschließend mit wäßrigen Spritzbrühen, die 80% Wirkstoff und 20% Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70% relativer Luftfeuchte aufgestellt. Nach 8 Tagen wird das Ausmaß der Rostpilzentwicklung auf den Blättern ermittelt.

Das Ergebnis des Versuches zeigt, daß die Wirkstoffe 1, 18, 22, 27, 29, 32, 40, 56, 58, 60, 68 und 80 bei der Anwendung als 0,025% ige Spritzbrühen eine bessere fungizide Wirkung zeigen (97%) als das Vergleichsmittel (50%).

Anwendungsbeispiel 3

Wirksamkeit gegen Pyrenophora teres

Gerstenkeimlinge der Sorte "Asse" werden im Zweiblattstadium mit wäßrigenSuspensionen, die 80% Wirkstoff und 20% Emulgator in der Trockensubstanz enthalten, tropfnaß gespritzt. Nach 24 Stunden werden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend werden die Pflanzen im Gewächshaus bei 20 bis 22°C und 70% relativer Luftfeuchtigkeit für weitere 5 Tage kultiviert. Dann wird das Ausmaß der Symptomentwicklung ermittelt.

Das Ergebnis des Versuches zeigt, daß die Wirkstoffe 1, 2, 18, 22, 27, 40, 45, 56, 58, 60, 62, 68, 72, 73 und 86 bei der Anwendung als 0,05% ige Spritzbrühe eine bessere fungizide Wirkung zeigen (97%) als das Vergleichsmittel (70%).

Anwendungsbeispiel 4

Wirksamkeit gegen Septoria nodorum

Blätter von in Töpfen gewachsenen Weizenkeimlingen der Sorte "Jubilar" werden mit wäßriger Spritzbrühe, die 80% Wirkstoff und 20% Emulgiermittel in der Trockensubstanz enthält, bis zur Tropfnässe besprüht. Am folgenden Tag werden die angetrockneten Pflanzen mit einer wäßrigen Sporensuspension von Septoria nodorum infiziert und dann für 7 Tage bei 17 bis 19°C und 95% relativer Luftfeuchtigkeit weiter kultiviert. Das Ausmaß des Pilzbefalls wird dann visuell ermittelt.

Das Ergebnis des Versuches zeigt, daß die Wirkstoffe 18, 22, 29, 31, 32, 45, 58, 60, 68, 86, 91, 94, 98, 107 und 112 bei der Anwendung als 0,05% ige Spritzbrühe eine bessere fungizide Wirkung zeigen (97%) als der Vergleichswirkstoff (50%).

## Ansprüche

1. Aryloxycarbamoylazole der Formel (I)

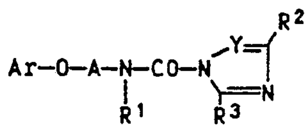

(I),

worin Ar Phenyl oder einen durch Halogen, Trifluormethyl, $C_1$- bis $C_4$-Alkyl, $C_1$- bis $C_6$-Alkoxy, Nitro oder Cyano substituierten Phenylrest bedeutet, und

$R^1$ einen durch Fluor, Chlor oder Brom substituierten Alkylrest mit 1 bis 6 Kohlenstoffatomen bedeutet, und

A den Rest $-(CH_2)_n-$ mit n=1 bis 10 bedeutet,

Y CH oder N bedeutet, und

$R^2$ und $R^3$ gleich oder verschieden sind und Wasserstoff oder Alkyl mit 1 bis 5 C-Atomen bedeuten.

2. Verfahren zur Herstellung von Aryloxycarbamoylazolen der Formel (I) gemäß Anspruch 1, dadurch gekennzeichnet, daß man eine Verbindung der Formel (II)

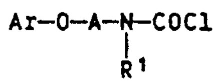

(II),

in der Ar, A und $R^1$ die im Anspruch 1 angegebenen Bedeutungen haben,

a) mit Azolen der Formel (III)

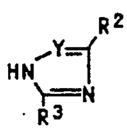

(III),

in der $R^2$, $R^3$ und Y die im Anspruch 1 angegebenen Bedeutungen haben, oder

b) mit deren Metall-Derivaten der Formel (IV),

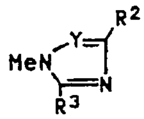 (IV),

in der $R^2$, $R^3$ und Y die im Anspruch 1 angegebenen Bedeutungen haben und Me Lithium, Natrium, Kalium oder Calcium bedeutet, oder

c) mit deren Silyl-Derivaten der Formel (V)

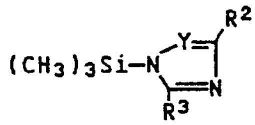 (V),

in der $R^2$, $R^3$ und Y die im Anspruch 1 angegebenen Bedeutungen haben, umsetzt.

3. Verfahren zur Herstellung von Aryloxycarbamoylazolen der Formel (I) gemäß Anspruch 1, dadurch gekennzeichnet, daß man eine Verbindung der Formel (VI)

$$Ar{-}O{-}A{-}NH{-}R^1 \qquad (VI),$$

in der Ar, $R^1$ und A die im Anspruch 1 angegebenen Bedeutungen haben, mit einem Carbonyl-bisazol der Formel (VII)

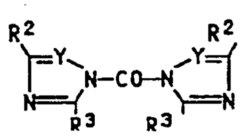 (VII),

in der $R^2$, $R^3$ und Y die im Anspruch 1 angegebenen Bedeutungen haben, umsetzt.

4. Fungizides Mittel, enthaltend einen festen oder flüssigen Trägerstoff und ein Aryloxy-carbamoylazol der Formel (I)

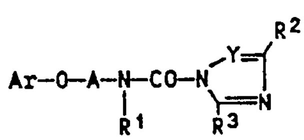 (I),

worin Ar Phenyl oder einen durch Halogen, Trifluormethyl, C1- bis $C_4$-Alkyl, $C_1$- bis $C_6$-Alkoxy, Nitro oder Cyano substituierten Phenylrest bedeutet, und

$R^1$ einen durch Fluor, Chlor oder Brom substituierten Alkylrest mit 1 bis 6 Kohlenstoffatomen bedeutet, und

A den Rest $-(CH_2)_n$ mit n=1 bis 10 bedeutet,

Y CH oder N bedeutet, und

$R^2$ und $R^3$ gleich oder verschieden sind und Wasserstoff oder Alkyl mit 1 bis 5 C-Atomen bedeuten.

5. Verfahren zur Herstellung von Fungiziden, dadurch gekennzeichnet, daß man eine Verbindung der Formel I gemäß Anspruch 1 mit festen oder flüssigen Trägerstoffen mischt.

6. Verfahren zur Bekämpfung von Fungi, dadurch gekennzeichnet, daß man ein Aryloxycarbamoylazol

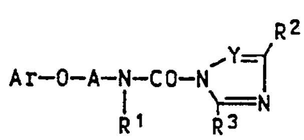 (I),

worin Ar Phenyl oder einen durch Halogen, Trifluormethyl, $C_1$- bis $C_4$-Alkyl, $C_1$- bis $C_6$-Alkoxy, Nitro oder Cyano substituierten Phenylrest bedeutet, und

$R^1$ einen durch Fluor, Chlor oder Brom substituierten Alkylrest mit 1 bis 6 Kohlenstoffatomen bedeutet, und

A den Rest $-(CH_2)_n-$ mit n=1 bis 10 bedeutet,

Y CH oder N bedeutet, und

$R^2$ und $R^3$ gleich oder verschieden sind und Wasserstoff oder Alkyl mit 1 bis 5 C-Atomen bedeuten, auf die Pilze oder durch Pilzbefall bedrohte Flächen, Materialien, Pflanzen oder Saatgüter einwirken läßt.

7. Verbindung gemäß Anspruch 1, dadurch gekennzeichnet, daß Ar Phenyl, A 1,4-Butylen, $R^1$ 4-Chlorbutyl, Y CH und $R^3$ Wasserstoff bedeuten.

8. Verbindung gemäß Anspruch 1, dadurch gekennzeichnet, daß Ar Phenyl, A 1,5-Pentylen, $R^1$ 4-Chlorbutyl, Y CH und $R^2$ und $R^3$ Wasserstoff bedeuten.

9. Verbindung gemäß Anspruch 1, dadurch gekennzeichnet, daß Ar Phenyl, A 1,6-Hexylen, $R^1$ 4-Chlorbutyl, Y CH und $R^2$ und $R^3$ Wasserstoff bedeuten.

## Claims

1. An aryloxycarbamoylazole of the formula (I)

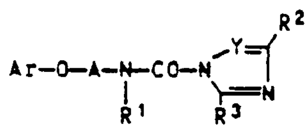

(I),

where Ar is phenyl which is unsubstituted or substituted by halogen, trifluoromethyl, $C_1$-$C_4$-alkyl, $C_1$-$C_6$-alkoxy, nitro or cyano, $R^1$ is alkyl having 1 to 6 carbon atoms which is substituted by fluorine, chlorine or bromine, A is $-(CH_2)_n-$ in which n is 1 to 10, Y is CH or N and $R^2$ and $R^3$ are identical or different and are each hydrogen or alkyl having 1 to 5 carbon atoms.

2. A process for the preparation of an aryloxycarbamoylazole of the formula (I) as claimed in claim 1, wherein a compound of the formula (II)

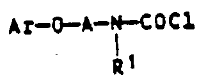

(II),

where Ar, A and $R^1$ have the meanings stated in claim 1, is reacted with

a) an azole of the formula (III)

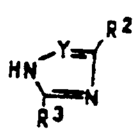

(III),

where $R^2$, $R^3$ and Y have the meanings stated in claim 1, or

b) with one of its metal derivatives of the formula (IV)

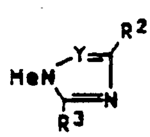

(IV),

where $R^2$, $R^3$ and Y have the meanings stated in claim 1 and Me is lithium, sodium, potassium or calcium, or

c) with one of its silyl derivatives of the formula (V)

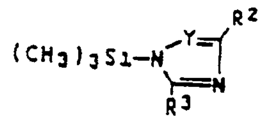

(V),

where $R^2$, $R^3$ and Y have the meanings stated in claim 1.

3. A process for the preparation of an aryloxycarbamoylazole of the formula (I) as claimed in claim 1, wherein a compound of the formula (VI)

$$Ar\text{-}O\text{-}A\text{-}NH\text{-}R^1 \qquad (VI),$$

where Ar, $R^1$ and A have the meanings stated in claim 1, is reacted with a carbonylbisazole of the formula (VII)

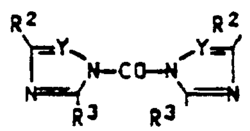

(VII),

where $R^2$, $R^3$ and Y have the meanings stated in claim 1.

4. A fungicide containing a solid or liquid carrier and an aryloxycarbamoylazole of the formula (I)

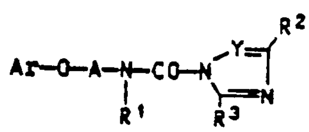

(I),

where Ar is phenyl which is unsubstituted or substituted by halogen, trifluoromethyl, $C_1$-$C_4$-alkyl, $C_1$-$C_6$-alkoxy, nitro or cyano, $R^1$ is alkyl having 1 to 6 carbon atoms which is substituted by fluorine, chlorine or bromine, A is $-(CH_2)_n-$ in which n is 1 to 10, Y is CH or N and $R^2$ and $R^3$ are identical or different and are each hydrogen or alkyl having 1 to 5 carbon atoms.

5. A process for the preparation of a fungicide, wherein a compound of the formula I as claimed in claim 1 is mixed with a solid or liquid carrier.

6. A method for controlling fungi, wherein an aryloxycarbamoylazole

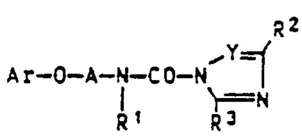

(I),

where Ar is phenyl which is unsubstituted or substituted by halogen, trifluoromethyl, $C_1$-$C_4$-alkyl, $C_1$-$C_6$-alkoxy, nitro or cyano, $R^1$ is alkyl having 1 to 6 carbon atoms which is substituted by fluorine, chlorine or bromine, A is $-(CH_2)_n-$ in which n is 1 to 10, Y is CH or N and $R^2$ and $R^3$ are identical or different and are each hydrogen or alkyl having 1 to 5 carbon atoms, is allowed to act on fungi or on surfaces, materials, plants or seeds which are threatened by fungal attacks.

7. A compound as claimed in claim 1, wherein Ar is phenyl, A is 1,4-butylene, $R^1$ is 4-chlorobutyl, Y is CH and $R^3$ is hydrogen.

8. A compound as claimed in claim 1, wherein Ar is phenyl, A is 1,5-pentylene, $R^1$ is 4-chlorobutyl, Y is CH and $R^2$ and $R^3$ are each hydrogen.

9. A compound as claimed in claim 1, wherein Ar is phenyl, A is 1,6-hexylene, $R^1$ is 4-chlorobutyl, Y is CH and $R^2$ and $R^3$ are each hydrogen.

## Revendications

1. Aryloxycarbamoylazoles de formule (I)

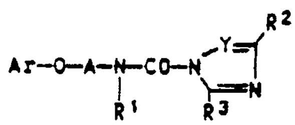

(I),

dans laquelle Ar représente phényle ou un reste phényle substitué par halogène, trifluorométhyle, alkyle en C1 à $C_4$, alcoxy en $C_1$ à $C_6$, nitro ou cyano, et

$R^1$, un reste alkyle à 1 à 6 atomes de carbone, substitué par fluor, chlore ou brome, et

A, le reste $-(CH_2)_n-$ avec n=1 à 10,

Y, CH ou N,

$R^2$ et $R^3$, qui peuvent être identiques ou différents, représentent hydrogène ou alkyle à 1 à 5 atomes C.

2. Procédé de préparation d'aryloxycarbamoyles de formule (I), selon la revendication 1, caractérisé par le fait que l'on met à réagir un composé de formule (II)

$$\underset{\underset{R^1}{|}}{Ar\text{-}O\text{-}A\text{-}N}\text{--}COCl \qquad (II),$$

dans laquelle Ar, A et $R^1$ ont les significations données dans la revendication 1,

a) avec des azoles de formule (III)

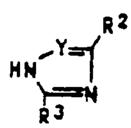

(III),

dans laquelle $R^2$, $R^3$ et Y ont les significations données dans la revendication 1, ou

b) avec leurs dérivés métalliques de formule (IV),

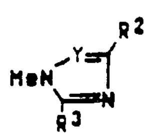

(IV),

dans laquelle $R^2$, $R^3$ et Y ont les significations données dans la revendication 1 et Me représente lithium, sodium, potassium ou calcium, ou

c) avec leurs dérivés de silyle de formule (V)

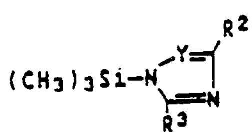

(V),

dans laquelle $R^2$, $R^3$ et Y ont les significations données dans la revendication 1.

3. Procédé de préparation d'aryloxycarbamoylazoles de formule (I), selon la revendication 1, caractérisé par le fait que l'on fait réagir un composé de formule (VI)

$$Ar-O-A-NH-R^1 \qquad (VI),$$

dans laquelle Ar, $R^1$ et A ont les significations données dans la revendication 1, avec un carbonyl-bisazole de formule (VII)

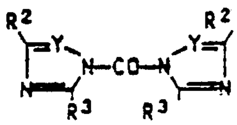

(VII),

dans laquelle $R^2$, $R^3$ et Y ont les significations données dans la revendication 1.

4. Fongicide, contenant un véhicule solide ou liquide et un aryloxycarbamoylazole de formule (I)

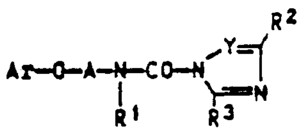

(I),

dans laquelle Ar représente phényle ou un reste phényle substitué par halogène, trifluorométhyle, alkyle en $C_1$ à $C_4$, alcoxy en $C_1$ à $C_6$, nitro ou cyano,

$R^1$, un reste alkyle à 1 à 6 atomes de carbone, substitué par fluor, chlore ou brome ?

A, le reste $-(CH_2)_n$, avec n=1 à 10,

Y, CH ou N et

$R^2$ et $R^3$, qui peuvent être identiques ou différents, représentent hydrogène ou alkyle à 1 à 5 atomes C.

5. Procédé de préparation de fongicides, caractérisé par le fait que l'on mélange un composé de formule I, selon la revendication 1, avec des véhicules solides ou liquides.

6. Procédé de lutte contre des champignons, caractérisé par le fait que l'on fait agir sur les champignons ou les surfaces, matériaux, plantes ou semences menacés par une attaque par les champignons, un aryloxycarbamoylazole

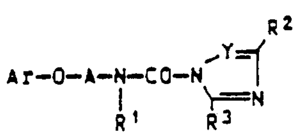

(I),

dans laquelle Ar représente phényle ou un reste phényle substitué par halogène, trifluorométhyle, alkyle en $C_1$ à $C_4$, alcoxy en $C_1$ à $C_6$, nitro ou cyano, et

$R^1$, un reste alkyle à 1 à 6 atomes de carbone, substitué par fluor, chlore ou brome, et

A, le reste $-(CH_2)_n-$ avec n=1 à 10,

Y, CH ou N,

$R^2$ et $R^3$, qui peuvent être identiques ou différents, représentent hydrogène ou alkyle à 1 à 5 atomes C.

7. Composé selon la revendication 1, caractérisé par le fait que Ar représente phényle, A, 1,4-butylène, $R^1$, 4-chlorobutyle, Y, CH et $R^2$ et $R^3$, hydrogène.

8. Composé selon la revendication 1, caractérisé par le fait que Ar représente phényle, A, 1,5-pentylène, $R^1$, 4-chlorobutyle, Y, CH et $R^2$ et $R^3$, hydrogène.

9. Composé selon la revendication 1, caractérisé par le fait que Ar représente phényle, A, 1,6-hexylène, $R^1$, 4-chlorobutyle, Y, CH et $R^2$ et $R^3$, hydrogène.